(19) Europäisches Patentamt
European Patent Office
Office européen des brevets

(11) **EP 0 988 270 B1**

(12) **EUROPÄISCHE PATENTSCHRIFT**

(45) Veröffentlichungstag und Bekanntmachung des Hinweises auf die Patenterteilung:
**19.03.2003 Patentblatt 2003/12**

(21) Anmeldenummer: **98934898.2**

(22) Anmeldetag: **26.05.1998**

(51) Int Cl.[7]: **C07C 45/00**, C07C 47/04

(86) Internationale Anmeldenummer:
**PCT/EP98/03083**

(87) Internationale Veröffentlichungsnummer:
**WO 98/055435 (10.12.1998 Gazette 1998/49)**

(54) **VERFAHREN ZUR NICHT OXIDATIVEN HERSTELLUNG VON FORMALDEHYD AUS METHANOL**

METHOD FOR PRODUCING FORMALDEHYDE FROM METHANOL IN A NON-OXIDATING MANNER

PROCEDE POUR LA PRODUCTION NON OXYDATIVE DE FORMALDEHYDE A PARTIR DE METHANOL

(84) Benannte Vertragsstaaten:
**BE DE FR GB IT NL**

(30) Priorität: **02.06.1997 DE 19722774**
**30.06.1997 DE 19727519**
**30.06.1997 DE 19727520**
**30.09.1997 DE 19743145**
**31.03.1998 DE 19814284**

(43) Veröffentlichungstag der Anmeldung:
**29.03.2000 Patentblatt 2000/13**

(73) Patentinhaber: **Ticona GmbH**
**65451 Kelsterbach (DE)**

(72) Erfinder:
- **SCHWEERS, Elke**
**D-69812 Bad Soden (DE)**
- **SCHULZ, Rolf**
**D-65931 Frankfurt (DE)**
- **KAISER, Thomas**
**D-65779 Kelkheim (DE)**
- **DINGERDISSEN, Uwe**
**D-64342 Seeheim-Jugenheim (DE)**

(56) Entgegenhaltungen:
**EP-A- 0 494 350** **EP-A- 0 691 338**
**DE-A- 2 525 174**

- **J. SAUER ET AL.: "The Catalyzed Dehydrogenation of Methanol to Formaldehyd at High Temperatures" CHEM. ENG TECHNOL., Bd. 18, 1995, Seiten 284--291, XP002077501 in der Anmeldung erwähnt**
- **FALBE, J.; REGITZ, M.: "Römpp-Lexikon Chemie", 1997, GEORG THIEME VERLAG, STUTTGART**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Mehrere Verfahren zur Herstellung von Formaldehyd aus Methanol sind bekannt (siehe z.B. Ullmann's Encyclopaedia of Industrial Chemistry). Technisch durchgeführt werden überwiegend die Oxidation

$$CH_3OH + \frac{1}{2} O_2 \rightarrow CH_2O + H_2O$$

an Eisen- und Molybdänoxyd enthaltenden Katalysatoren bei 300°C bis 450°C (Formox Prozeß) und die oxidative Dehydrierung (Silberkontakt-verfahren) gemäß:

$$CH_3OH \rightarrow CH_2O + H_2$$

$$H_2 + \frac{1}{2} O_2 \rightarrow H_2O$$

bei 600°C bis 720°C. Nach beiden Verfahren liegt der Formaldehyd zunächst als wäßrige Lösung vor. Insbesondere bei der Verwendung für die Herstellung von Formaldehyd-Polymeren und -Oligomeren muß der so gewonnene Formaldehyd aufwendig entwässert werden. Ein weiterer Nachteil ist die Bildung korrosiver, die Polymerisation negativ beeinflussender Ameisensäure als Nebenprodukt.

Durch die Dehydrierung von Methanol können diese Nachteile vermieden und kann im Gegensatz zu oben genannten Verfahren nahezu wasserfreier Formaldehyd direkt gewonnen werden:

$$CH_3OH \xrightarrow{\text{Kat.}} CH_2O + H_2$$

Um ein ökologisches und wirtschaftlich interessantes technisches Verfahren für die Dehydrierung von Methanol zu erreichen, sollten die folgenden Voraussetzungen erfüllt werden: Die stark endotherme Reaktion sollte bei hohen Temperaturen durchgeführt werden, damit hohe Umsätze erreicht werden. Konkurrierende Nebenreaktionen müssen unterdrückt werden, um eine hinreichende Selektivität für Formaldehyd zu erzielen (unkatalysiert beträgt die Selektivität für die Bildung von Formaldehyd bei Umsätzen über 90 % weniger als 10 %). Die Verweilzeiten müssen kurz oder die Abkühlung der Reaktionsprodukte schnell sein, um den Zerfall des thermodynamisch bei den Reaktionsbedingungen nicht stabilen Formaldehyds

$$CH_2O \rightarrow CO + H_2$$

zu verringern.

Verschiedene Verfahren zur Durchführung dieser Reaktion wurden vorgeschlagen; so ist beispielsweise in der DE-A-37 19 055 ein Verfahren zur Herstellung von Formaldehyd aus Methanol durch Dehydrierung in Gegenwart eines Katalysators bei erhöhter Temperatur beschrieben. Die Umsetzung wird in Gegenwart eines mindestens eine Natriumverbindung enthaltenden Katalysators bei einer Temperatur von 300°C bis 800°C durchgeführt.

J. Sauer und G. Emig (Chem. Eng. Technol. 1995, 18, 284-291) gelang es, aus einem $NaAlO_2$ und $LiAlO_2$ enthaltenden Katalysator durch ein reduzierendes Gasgemisch (87%$N_2$ + 13%$H_2$) eine katalytisch aktive Spezies freizusetzen, bei der es sich seiner Vermutung nach um Natrium handelt. Diese Spezies vermag die Dehydrierung von im selben Reaktor stromabwärts zugegebenem, d.h. nicht mit der Katalysatorschüttung in Kontakt gekommenem, Methanol zu Formaldehyd zu katalysieren. Bei Verwendung nicht reduzierender Gase wurde nur geringe Katalysatoraktivität gefunden.

Nach J. Sauer und G. Emig sowie Ergebnissen aus neueren Untersuchungen (siehe z.B. M. Bender et al., Vortrag auf dem XXX. Jahrestreffen deutscher Katalytiker, 21.-23.3.1997) wurden Natriumatome und NaO-Moleküle als in die Gasphase emittierte Verbindungen identifiziert und deren katalytische Aktivität für die Dehydrierung von Methanol in der Gasphase beschrieben.
Das Ausgangsmaterial Methanol wird bei den bekannten Verfahren stets verdünnt mit Stickstoff und/oder Stickstoff/Wasserstoff-Gemischen umgesetzt.

Obwohl mit den bekannten Verfahren bereits gute Ergebnisse erzielt werden, besteht doch ein breiter Raum

für Verbesserungen in technischer und ökonomischer Hinsicht, besonders weil sich die eingesetzten Katalysatoren mit der Zeit verbrauchen oder inaktivieren sowie die Formaldehydausbeuten noch verbesserungsfähig sind.

Es wurde überraschend gefunden, daß es möglich ist, die Ausbeute der Dehydrierung von Methanol durch eine verbesserte Reaktionsführung zu erhöhen. Dies kann erreicht werden, indem in den Bereichen Primärkatalysatorzersetzung und Reaktionsteil getrennte Temperaturen und gegebenenfalls auch Verweilzeiten eingestellt werden, insbesondere wenn das Temperaturniveau im eigentlichen Reaktionsteil niedriger als in der Primärkatalysatorzugabeeinheit eingestellt wird.

Auf diese Art und Weise lassen sich Methanolumsätze von über 95 % und hohe Formaldehydselektivitäten erreichen und überraschend auch nicht reduzierende Gase als Trägergas verwenden.

Gegenstand der Erfindung ist daher ein Verfahren zur Herstellung von Formaldehyd aus Methanol durch Dehydrierung in einem Reaktor in Gegenwart eines Katalysators bei einer Temperatur im Bereich von 300 bis 1000°C, dadurch gekennzeichnet, daß man die Erzeugung des Katalysators räumlich getrennt vom Reaktor und bei einer Temperatur oberhalb der Dehydrierungstemperatur durchführt.

Vorteile einer tieferen Reaktionstemperatur liegen in dem geringeren energetischen und apparativen Aufwand zur Erwärmung/Abkühlung vor/nach der Reaktion, der geringen Zerfallsgeschwindigkeit des bei Reaktionsbedingungen thermisch instabilen Formaldehyds und den geringeren Anforderungen an die Werkstoffe.

Bevorzugt beträgt die Temperaturdifferenz mindestens 20°C, besonders bevorzugt 40 bis 250°C.

Aus geeigneten Primärkatalysatoren werden bei thermischer Behandlung in der Primärkatalysatorzersetzungszone und beim Überströmen mit einem reduzierenden oder auch mit einem nicht reduzierendem Gas, wie molekularem Stickstoff, bei Temperaturen, die nicht gleich der Reaktionstemperatur für die Dehydrierung, sondern höher sind, eine oder mehrere katalytisch aktive Spezies ausgetragen bzw. erzeugt und/oder auf ihm erzeugt (Sekundärkatalysator), die in der Lage sind, die Dehydrierung von Methanol zu katalysieren. Ein solch fluider Katalysator kann über erhebliche Strecken transportiert werden, ohne einen merklichen Verlust an Wirksamkeit in der Dehydrierung zu erleiden. Diese getrennte Temperatureinstellung erlaubt durch Anpassung an die jeweiligen Bedingungen zur Katalysatorfreisetzung/-verdampfung bzw. Erzeugung einer katalytisch aktiven Spezies (Sekundärkatalysator) einerseits und zur Reaktion andererseits insbesondere die Möglichkeit zur Erniedrigung der Reaktionstemperatur. Damit vermindert sich der Zerfall des instabilen Formaldehyds durch Folgereaktionen und erhöht sich die Ausbeute.

Bevorzugte Temperaturen zur Erzeugung der katalytisch aktiven Spezies aus dem Primärkatalysator liegen zwischen 300 und 1100°C, besonders bevorzugt sind Temperaturen zwischen 400 und 1000°C.

Bevorzugte Temperaturen zur Dehydrierung des Methanols liegen zwischen 200 und 1000°C, besonders bevorzugt sind Temperaturen zwischen 300 und 980°C.

Weiterhin können die Verweilzeiten im Dehydrierungsreaktor und Behälter zur Primärkatalysatorzugabe bzw. zur Erzeugung des Sekundärkatalysators über die Aufteilung des Trägergasstromes getrennt eingestellt werden. Dadurch wird eine gezielte Beladung des durch die Katalysatorzugabeeinheit geleiteten Gasstromes mit der aktiven Spezies erreicht.

Bevorzugte Verweilzeiten zur Erzeugung des Sekundärkatalysator liegen zwischen 0,01 und 60 sec, besonders bevorzugt zwischen 0,05 und 3 sec, ganz besonders bevorzugt 0,05 und 1 sec. Zur Dehydrierung des Methanols sind Verweilzeiten in der Reaktionszone von 0,005 bis 30 sec bevorzugt, besonders bevorzugt 0,01 bis 15 sec, ganz besonders bevorzugt 0,05 bis 3 sec.

Durch das Ersetzen des verbrauchten Primärkatalysators wird ein kontinuierliches Verfahren zur nicht oxidativen Methanoldehydrierung möglich, das verbesserte Methanolnutzung bzw. Formaldehydausbeuten liefert.

Die Trägergasströme können aus einem reduzierenden oder nicht reduzierendem Gas, wie z.B. $H_2$/CO -Gemischen oder Stickstoff, vorzugsweise aus den Nebenprodukten der Dehydrierung bestehen.

Figur 1 gibt einen schematischen Überblick über eine bevorzugte Variante des erfindungsgemäßen Verfahrens.

Der Trägergasstrom 1 wird im Wärmetauscher 2 erwärmt und in einen oder mehrere beheizte Behälter 3, in denen die aktive Katalysatorspezies erzeugt wird, geleitet. Aus einem Vorratsgefäß 4 wird Primärkatalysator 5 ebenfalls in den Behälter 3 gefördert. Der Austrittsstrom aus Behälter 3 wird in den Reaktor 6 geleitet. Aus einem Vorratsgefäß 7 wird Methanol 8 gefördert, in einem Wärmetauscher 9 erwärmt und in den Reaktor 6 geführt. Die Produktgase aus dem Reaktor 6 werden im Wärmetauscher 10 abgekühlt und einer Einheit 27 zum Abtrennen des Formaldehyds zugeführt.

Weiterhin Gegenstand der Erfindung ist daher eine Vorrichtung zur Durchführung des obengenannten Verfahrens enthaltend einen oder mehrere Wärmeaustauscher zum Vorwärmen der Ausgangsstoffe, einen beheizten Behälter zur Zersetzung des Primärkatalysators, einen beheizten Reaktor zur Durchführung der Dehydrierung, einen oder mehrere Wärmeaustauscher zum Abkühlen des Produktgemisches, eine Einrichtung zum Abtrennen des Formaldehyds, eine Vorrichtung zum Einbringen des Methanols und zur Nachführung des Primärkatalysators, sowie Mittel zur Einstellung unterschiedlicher Temperaturen in dem Behälter zur Zersetzung des Primärkatalysators und dem Reaktor.

Der Primärkatalysator kann als Feststoff, in einem Lösungsmittel gelöst, als Flüssigkeit oder als Schmelze jeweils kontinuierlich oder diskontinuierlich nachgeführt werden.

Ein Vorteil der kontinuierlichen oder diskontinuierlichen Dosierung oder Nachführung des Primärkatalysators ist eine vom ursprünglich zugegeben Katalysatorausgangsmaterial unabhängige und wesentlich verlängerte Betriebsdauer der Anlage. Ein weiterer Vorteil der getrennten Zugabe liegt außerdem in einem gezielten und definierten Eintrag der aktiven Substanz sowie der Möglichkeit einer gleichmäßigen Bereitstellung der katalytisch aktiven Spezies.

Die Nachführung des Primärkatalysators als Feststoff, z.B. pulverförmig, körnig oder kompaktiert, erfolgt über eine Feststoffdosierung, z.B. mit Hub- oder Dreh-Kolben, Zellradschleuse, Schnecke oder Schüttelrinne.

Wird der Primärkatalysator, z.B. Natrium oder eine Natriumverbindung, gelöst zugegeben, sind besonders Lösungsmittel mit einer chemischen Zusammensetzung geeignet, die nur die im Prozeß schon vorhandenen Elemente (C,H,O) enthalten. Besonders bevorzugt wird MeOH als Lösungsmittel. Die Zugabe erfolgt z.B. über eine Düse, die gekühlt werden kann, um ein Verdampfen des Lösungsmittels, Auskristallisieren oder Ablagerungen des festen Primärkatalysators in der Düse zu vermeiden.

Die Zugabe des Primärkatalysators als Schmelze ist z.B. über eine Düse möglich. Die Schmelze kann dann direkt im Gasstrom verdampft oder zersetzt werden.

Bei allen Möglichkeiten der Primärkatalysatornachführung geschieht dies vorteilhaft in einer Art und Weise, daß das Material in intensivem Kontakt mit strömendem Gas steht. Dies kann beispielsweise durch Aufbringen des Katalysatormaterials nach den oben beschriebenen Verfahren auf eine geeignete Oberfläche erreicht werden, die vom Gas durch - oder überströmt werden. Es kann sich hierbei um die Oberfläche eines Trägermaterials handeln, das in einem Festbett als Schüttung angeordnet ist. Als Materialien eignen sich z.B. SiC, $SiO_2$, $Al_2O_3$ etc. in einer geeigneten geometrischen Form, z.B. als Granulat, Pellets oder Kugeln. Die Anordnung geschieht vorzugsweise senkrecht in einem Festbett, vorzugsweise mit Dosierung von oben. Die eingetragene Substanz schlägt sich auf dem Trägermaterial nieder und die katalytisch aktive Substanz geht während des Prozesses in die Gasphase über.

Eine andere Möglichkeit ist die Anordnung des Primärkatalysators in einer Wirbelschicht, durch die der Trägergasstrom geleitet wird. Das Wirbelgut besteht dabei zumindest teilweise aus dem geträgerten oder ungeträgerten Primärkatalysator. Der Verlust an aktiver Substanz kann durch Nachführung von frischem Primärkatalysator ersetzt werden, verbrauchtes Material kann gegebenenfalls abgezogen werden. Realisiert werden kann dies im kontinuierlichen Fall z.B. durch eine zirkulierende Wirbelschicht.

Die Nachführung eines Primärkatalysators kann auch durch abwechselnde Sekundärkatalysatorerzeugung in verschiedenen Behältern erfolgen, in denen der Primärkatalysator beispielsweise als Festbett oder Wirbelschicht, jeweils geträgert oder ungeträgert, angeordnet sein kann.

Der Vorteil der Verwendung mehrerer Einheiten zur diskontinuierlichen Katalysatornachführung besteht darin, daß auch solche Primärkatalysatoren eingesetzt werden können, bei denen, z.B. aufgrund von Stoffeigenschaften wie z.B. Schmelzpunkt, Viskosität oder Zersetzungstemperatur, eine kontinuierliche Förderung nicht oder nur mit großem Aufwand möglich wäre.

Verbindungen, die in dem erfindungsgemäßen Verfahren eingesetzt werden können, sind beispielsweise aus der Literatur bekannte Katalysatoren , wie sie z.B. in Chem. Eng. Technol. 1994, 17, 34 beschrieben sind.

An Metallen eignen sich beispielsweise Li, Na, K, Cs, Mg, Al, In, Ga, Ag, Cu, Zn, Fe, Ni, Co, Mo, Ti, Pt, bzw. deren Verbindungen. Weiterhin geeignet sind beispielsweise S, Se, Phosphate von Übergangsmetallen, wie V und Fe, und Heteropolysäuren, wie Molybdatophosphorsäure.

Beispiele für konkrete Katalysatoren sind:

- Natrium oder Natriumverbindungen (DE-A-37 19 055 und DE-A-38 11 509)
- Aluminiumoxid, Alkalialuminat und/oder Erdalkalialuminat (EP-A-04 05 348)
- Silberoxid (JP-A 60/089 441, Dervent Report 85 - 15 68 91/26)
- ein Kupfer, Zink und Schwefel enthaltender Katalysator (DE-A 25 25 174)
- ein Kupfer, Zink und Selen enthaltender Katalysator (US-A 4,054,609)
- ein Zink und/oder Indium enthaltender Katalysator (EP-A 0 130 068)
- Silber (US-A 2,953,602)
- Silber, Kupfer und Silicium (US-A 2,939,883).
- Zink, Cadmium, Selen, Tellur oder Indium enthaltende Verbindungen.

Bevorzugt ist die Verwendung von Natrium oder Natriumverbindungen.

Die Anwendungsform eines solchen, beispielsweise natriumhaltigen Katalysators kann in weitem Rahmen variieren:

Metallisch, z.B. auch als Legierung mit mindestens einem anderen Legierungsbestandteil, als Verbindung oder Salz, wobei mindestens ein nichtmetallisches Element mit Na gebunden vorliegt (binäre Verbindungen und Salze). Ist mehr als ein Element in der Verbindung gebunden, so liegen binäre, ternäre oder quarternäre Verbindungen und

Salze vor. Ebenso bevorzugt ist der Einsatz in geträgerter Form, beispielsweise auf einem anorganischen Träger.

Wird Natrium metallisch eingesetzt, so kann es fest, flüssig oder bevorzugt dampfförmig eingesetzt werden. Bevorzugte Legierungen sind solche mit anderen Alkalimetallen und/oder Erdalkalimetallen, wie Ba, Sr, Ca, Cs, Rb, K oder besonders bevorzugt Li und/oder Magnesium.

Darüber hinaus können auch Legierungen mit B, Al, Si und Sn Verwendung finden. Dies gilt besonders auch für Legierungen, die Verbindungen wie Natriumborid, $NaB_2$, Natriumsilicid, NaSi oder NaSn enthalten können.

Geeignete binäre Na-Verbindungen und Salze sind beispielsweise Natriumcarbide, wie $Na_2C_2$, $NaC_8$, Natriumhalogenide, wie NaF, Natriumoxide, wie $Na_2O$, Natriumazid, Natriumphosphid, Natriumsulfid, Natriumpolysulfide, bevorzugt auch Natriumhydride, wie NaH.

Geeignete ternäre Na-Verbindungen und Salze sind beispielsweise Natriumborate, wie Borax, Natriumphosphate bzw. -hydrogenphosphate, Natriumphosphite, Natrium(meta)silikate und -alumosilikate, wie Wasserglas, $Na_3AlF_6$ (Kryolith), Natrium(hydrogen)sulfat, Natriumsulfit, Natriumnitrit, Natriumnitrat, Natriumamid, Natriumacetylid NaCCH, Natriumcyanid, Natriumrhodanid, Natriumthiomethylat, Natriumthiosulfat, bevorzugt jedoch NaOR, mit R = H, organischer Rest (= Salze organischer Säuren, Alkoholate, Phenolate, Acetylacetonat, Acetessigsäureestersalz, Salze der Salicylsäure oder des Salicylaldehyds), Natriumcarbonat und Natriumhydrogencarbonat und deren Gemische, wie Soda, Thermonatrit, Trona, Pirssonit, Natrocalcit. Generell ist der Einsatz wasserfreier, d.h. getrockneter Salze vorzuziehen.

Besonders bevorzugt sind NaOH, NaOOC-R' (vorzugsweise Formiat, Acetat, Lactat, Oxalat), NaOR' (R' ist ein organisches Radikal mit 1 bis 4 C-Atomen) und Natriumcarbide.

Ganz besonders bevorzugt sind NaOH, Natriumformiat, Natriummethylat, Natriumacetat und Natriumcarbide, wie $Na_2C_2$.

Geeignete quarternäre Verbindungen sind z.B. natriumhaltige Alumosilikate, die künstlich hergestellt werden können oder auch vielfältig als natürliche Mineralien und Gesteine (z.B. Natronfeldspat oder Albit und Kalk-Natronfeldspat oder Oligoklas) vorkommen. Sie können zusätzlich durch Ionenaustausch mit Na beladen werden.

Vorteilhaft können auch Doppelsalze vom Typ des Alauns oder Thenardit, Glauberit, Astrakanit, Glaserit, Vanthoffit Verwendung finden.

Die hier genannten Natriumverbindungen und Salze können vorteilhaft auch als Gemische vorliegen. Insbesondere sind durchaus auch Gehalte von < 50%, bevorzugt < 30% von Kationen anderer Alkalimetalle und/oder Erdalkalimetalle, wie Ba, Sr, Ca, Cs, Rb, K oder bevorzugt Li und / oder Magnesium einsetzbar. Besonders vorteilhaft sind technisch erhältliche, komplexe Gemische, wie Natronkalk, Thomasmehl und Zemente, z.B. Portlandzement, gegebenenfalls nach Anreicherung mit Natrium durch Lagerung in natriumhaltigen Lösungen (NaCl, Meerwasser).

Besonders vorteilhaft werden zur Herstellung des fluiden Katalysators solche Verbindungen thermisch behandelt, die außer Natrium nur die im Prozeß bereits vorhandenen Elemente C, O und H besitzen, um im Prozeß weitgehend rückstandsfrei verbraucht zu werden. Besonders vorteilhaft ist es, daß so auch technische Verbindungen als Katalysator eingesetzt werden können.

Generell ist der Einsatz wasserfreier, d.h. getrockneter Verbindungen vorzuziehen.

Insbesondere bevorzugte Primärkatalysatoren sind Natriumverbindungen aus der Gruppe:

a) Natriumalkoholate,
b) Natriumcarboxylate,
c) Natriumsalze C-H acider Verbindungen und
d) Natriumoxid, Natriumhydroxid, Natriumnitrit, Natriumacetylid, Natriumcarbid, Natriumhydrid und Natriumcarbonyl.

Obengenannte Verbindungen liefern in dem erfindungsgemäßen Verfahren Formaldehyd Ausbeuten von über 60% und geringen Wasserkonzentrationen von weniger als 5 mol % $H_2O$ pro mol Formaldehyd bei Reaktionstemperaturen von 600 bis 1000°C.

Geeignete Reaktoren sind dem Fachmann bekannt und geläufig. Grundsätzlich können Reaktortypen und Aufbauten verwendet werden, wie sie aus der Literatur für Dehydrierungsreaktionen bekannt sind, wobei den korrosiven Eigenschaften gegebenenfalls im Fluidstrom enthaltener Substanzen Rechnung zu tragen ist. Solche Apparaturen sind beispielsweise in Winnacker/Küchler, Chemische Technologie, 4. Auflage, Kapitel "Technik der Pyrolyse" Hanser Verlag, München 1981-86, beschrieben.

Geeignete Reaktormaterialien sind z.B. keramische Werkstoffe, wie Korund, aber auch aufkohlungs-, temperatur- und zunderbeständige Eisen- und Nickelbasislegierungen, z.B. Inconel 600® und Hasteloy®. Wird eine Verbrennung zur Beheizung des Behälters 3 und/oder des Reaktors 6 verwendet, eignen sich hierfür z.B. von außen befeuerte Rohre.

Die dem Prozeß zuzuführende Wärme wird vorzugsweise durch die Verbrennung von Nebenprodukten der Dehydrierung, vornehmlich $H_2$ und CO, gewonnen.

Ebenso bevorzugt ist die Beheizung des Reaktors/Katalysatorbehälters durch Mikrowellen.

Für die Reaktion kann handelsübliches Methanol eingesetzt werden, vorzugsweise sollte es wasserarm sein und keine Stoffe enthalten, die den Katalysator vergiften.

Zur Durchführung der Dehydrierung wird das fluide, vorzugsweise gasförmige Methanol vorzugsweise mit Trägergas verdünnt.

Der molare Methanolanteil beträgt im allgemeinen 5 bis 90 %, vorzugsweise 10 bis 50 %, besonders bevorzugt 10 bis 40 %.

Der Druck ist bei dem erfindungsgemäßen Verfahren unkritisch. Die Dehydrierung des Methanols kann bei Unterdruck, Normaldruck oder Überdruck durchgeführt werden. Ein Bereich von etwa 0,1 bis 10 bar, vorzugsweise 0,5 bis 2 bar, ist besonders geeignet. Bevorzugt wird Normaldruck. Das erfindungsgemäße Verfahren kann diskontinuierlich oder kontinuierlich durchgeführt werden, wobei letzteres bevorzugt ist.

In einer bevorzugten Variante des erfindungsgemäßen Verfahrens wird dem Reaktor ein weiterer Trägergasstrom zugeführt, der eine Temperatur oberhalb, vorzugsweise mindestens 20°C, besonders bevorzugt 40 bis 250°C, der Dehydrierungstemperatur aufweist.

In einer bevorzugten Variante des erfindungsgemäßen Verfahrens führt man einen Kreisgasstrom, der aus Nebenprodukten der Dehydrierung besteht, als Trägergas durch die Reaktoren. Dieser Kreisgasstrom wird erhalten, in dem man nach Abtrennung des Formaldehyds die Nebenprodukte der Dehydrierung, vornehmlich $H_2$ und CO, mittels einer geeigneten Vorrichtung zumindest teilweise in den Reaktor zurückgeführt.

Die Abtrennung des Formaldehyds aus dem Reaktionsgemisch kann nach an sich bekannten, dem Fachmann geläufigen Methoden erfolgen, beispielsweise durch Polymerisation, Kondensation oder physikalische oder chemische Ab- oder Adsorption.

Eine technisch erprobte Methode ist die Bildung von Halbacetalen aus Formaldehyd und einem Alkohol. Die Halbacetale werden daran anschließend thermisch gespalten, wobei sehr reiner Formaldehyd-Dampf entsteht. Als Alkohol wird meist Cyclohexanol verwendet, da dessen Siedepunkt genügend weit über der Zersetzungstemperatur des Halbacetals liegt. Die Halbacetale werden üblicherweise in Fallfilm- oder Dünnschichtverdampfern bei Temperaturen von 100 bis 160°C gespalten (siehe z.B. US 2,848,500 vom 19.08.1958 "Preparation of Purified Formaldehyde" und

US 2,943,701 vom 05.07.1960 "Process for purification of gaseous formaldehyde", oder JP-A 62/289 540). Die dabei freiwerdenden Formaldehyd-Dämpfe enthalten noch geringe Mengen Verunreinigungen, die meist durch eine Gegenstromwäsche mit Alkohol, wie Cyclohexanolhemiformal, durch Kondensation oder auch durch gezielte Präpolymerisation, entfernt werden.

Besonders bevorzugte Methoden zur Reinigung des erfindungsgemäß hergestellten Formaldehyds sind in den deutschen Patentanmeldungen 19 747 647.3 und 19 748 380.1 beschrieben.

Eine weitere Methode zur Abtrennung von Formaldehyd aus dem Reaktionsgemisch ist die Bildung von Trioxan in einem katalytischen Gasphasenprozeß (siehe z.B. Appl. Catalysis A 1997, 150, 143-151 und EP-A 0 691 338). Trioxan kann dann z.B. auskondensiert werden.

Verwertungsmöglichkeiten für die Nebenprodukte der Reaktion , insbesondere Wasserstoff, sind beispielsweise die Synthese von Methanol oder die Gewinnung von reinem Wasserstoff, der z.B. durch Membranen abgetrennt werden kann.

So gewonnener Wasserstoff eignet sich beispielsweise zur Synthese von Ammoniak, in Raffinerieprozessen zur Herstellung von Benzin und Krackprodukten der Petrochemie, zur Methanolsynthese, zur Fetthärtung u.a. Hydrierungen, als Reduktionsmittel zur Gewinnung von W, Mo, Co u.a. Metallen, als reduzierendes Schutzgas bei metallurgischen Prozessen, zum autogenen Schweißen und Schneiden, als Brenngas in Mischung mit anderen Gasen (Stadtgas, Wassergas), oder verflüssigt als Treibstoff in Luft- und Raumfahrt.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Desinfektion und als Konservierungsmittel, ebenso als Zwischenprodukt zur Herstellung von methanolischen Formaldehydlösungen, Methylal, Kunststoffen, beispielsweise Polyoxymethylenen, Polyacetalen, Phenolharzen, Melaminen, Aminoplasten, Polyurethanen und Caseinkunststoffen, sowie 1,4-Butanol, Trimethylolpropan, Neopentylglykol, Pentaerythrit und Trioxan zur Herstellung von Farbstoffen, wie Fuchsin, Acridin, zur Herstellung von Düngemitteln sowie zur Behandlung von Saatgut.

Da Formaldehyd nach dem erfindungsgemäßen Verfahren üblicherweise mit geringem Wassergehalt hergestellt wird, eignet sich so hergestellter Formaldehyd insbesondere für die Polymerisation zu Polyoxymethylen und die Trimerisierung zu Trioxan, da hier wasserfreier Formaldehyd einzusetzen ist.

Die Erfindung betrifft auch derartig hergestellte Kunststoffe, wie Polyoxymethylen und Polyacetale, Trioxan, Farbstoffe, Düngemittel und Saatgut.

Weiterhin Gegenstand der Erfindung ist ein Verfahren zur Herstellung von Trioxan, dadurch gekennzeichnet, daß man

1. Methanol durch Dehydrierung in einem Reaktor bei einer Temperatur im Bereich von 300 bis 1000°C in Gegenwart eines Katalysators zu Formaldehyd umsetzt, wobei man die Erzeugung des Katalysators räumlich getrennt vom Reaktor und bei einer Temperatur oberhalb der Dehydrierungstemperatur durchführt, und
2. den so hergestellten Formaldehyd gegebenenfalls reinigt und zu Trioxan trimerisiert.

Einzelheiten der Herstellung von Trioxan sind dem Fachmann bekannt und geläufig. Sie sind z.B. in Kirk u. Othmer, Encyclopedia of Chemical Technology, 2. Aufl., Band 10, S. 83, 89, New York Interscience 1963-1972, beschrieben.
Gegenstand der Erfindung ist ebenso ein Verfahren zur Herstellung von Polyoxymethylen, dadurch gekennzeichnet, daß man

1. Methanol durch Dehydrierung in einem Reaktor bei einer Temperatur im Bereich von 300 bis 1000°C in Gegenwart eines Katalysators zu Formaldehyd umsetzt, wobei man die Erzeugung des Katalysators räumlich getrennt vom Reaktor und bei einer Temperatur oberhalb der Dehydrierungstemperatur durchführt, und
2. den so gewonnenen Formaldehyd gegebenenfalls reinigt,
3. den Formaldehyd polymerisiert,
4. die Endgruppen des so hergestellten Polymers absättigt (Capping) und
5. gegebenenfalls das Polymer in der Schmelze homogenisiert und/oder mit geeigneten Zusätzen versieht.

Die Herstellung von Polyoxymethylen aus Formaldehyd ist dem Fachmann bekannt und geläufig. Einzelheiten finden sich z.B. in Ullmann's Encyclopedia of Industrial chemistry, Bd. 21, 5. Aufl., Weinheim 1992, und der dort zitierten Literatur.
Ein weiterer Gegenstand der Erfindung ist ein Verfahren zur Herstellung von Polyoxymethylen Copolymeren, dadurch gekennzeichnet, daß man

1. Methanol durch Dehydrierung in einem Reaktor bei einer Temperatur im Bereich von 300 bis 1000°C in Gegenwart eines Katalysators zu Formaldehyd umsetzt, wobei man die Erzeugung des Katalysators räumlich getrennt vom Reaktor und bei einer Temperatur oberhalb der Dehydrierungstemperatur durchführt, und
2. den so gewonnenen Formaldehyd zu Trioxan trimerisiert,
3. gegebenenfalls das Trioxan reinigt,
4. das Trioxan mit cyclischen Ethem oder cyclischen Acetalen copolymerisiert,
5. gegebenenfalls instabile Endgruppen entfernt und
6. das so hergestellte Copolymer gegebenenfalls in der Schmelze homogenisiert und/oder mit geeigneten Zusatzstoffen versetzt.

Ein weiterer Gegenstand der Erfindung ist ein Verfahren zur Herstellung Polyoxymethylen Copolymeren, dadurch gekennzeichnet, daß man

1. Methanol durch Dehydrierung in einem Reaktor in Gegenwart eines Katalysators bei einer Temperatur im Bereich von 300 bis 1000°C zu Formaldehyd umsetzt, wobei man die Erzeugung des Katalysators räumlich getrennt vom Reaktor und bei einer Temperatur oberhalb der Dehydrierungstemperatur durchführt, und
2. den so gewonnenen Formaldehyd gegebenenfalls reinigt,
3. den Formaldehyd mit cyclischen Ethem oder cyclischen Acetalen copolymerisiert,
4. gegebenenfalls instabile Endgruppen entfernt und
5. das so hergestellte Polymer gegebenenfalls in der Schmelze homogenisiert und/oder mit geeigneten Zusatzstoffen versetzt.

Die Herstellung von Polyoxymethylen-Copolymeren ist dem Fachmann bekannt und geläufig. Einzelheiten finden sich beispielsweise in Ullmann's Encyclopedia of Industrial Chemistry, Bd. 21, 5. Aufl., Weinheim 1992 und der dort zitierten Literatur sowie in russischen Schriften SU 436067, 740715 und SU 72-1755156, 720303.
Auf den Inhalt der prioritätsbegründenden deutschen Patentanmeldungen 197 22 774.0 und 197 27 519.2 sowie auf die Zusammenfassung der vorliegenden Anmeldung wird ausdrücklich Bezug genommen. Sie gelten durch Zitat als Bestandteil der Beschreibung.
Die Erfindung wird durch die Beispiele näher erläutert, ohne sie dadurch beschränken zu wollen. Die Figur 2 zeigt dazu schematisch den Aufbau der Versuchsapparatur anhand eines verfahrenstechnischen Fließbildes.

Beispiele

Die Dehydrierung des Methanols wird in einem Rohrreaktor 26, der indirekt durch einen elektrischen Rohrofen 12 beheizt wird, durchgeführt. Eine Katalysatorzugabeeinheit wird durch ein Metallrohr 11 gebildet, das indirekt durch den elektrischen Rohrofen 12 beheizt wird. In dem Rohr 11 ist eine Schüttung 13 aus Trägermaterial angeordnet, auf der Primärkatalysator (0,1- 5,0 g) vorgelegt ist. In dieses Rohr 11 wird ein Teil 14 eines überhitzten Trägergasstromes 15 geleitet, der vorher über beheizte Zuleitungen vorgewärmt wurde. In dieses Rohr 11 wird außerdem Primärkatalysator als Lösung über eine Düse 16 nachgeführt. Der Primärkatalysator schlägt sich auf der Schüttung 13 nieder.
Der Trägergasteilstrom 14 wird durch die Schüttung geleitet, um ihn mit einer sich bildenden aktiven Katalysator-Spezies zu beladen.
Der Gesamtstrom wird anschließend in den Reaktionsraum 19 geleitet.
Methanol 17 wird vorgewärmt und in einem weiteren Teil 18 des Trägergasstromes 15 gefördert und ebenfalls dem Reaktionsraum 19 zugegeben.
Ein dritter Gasstrom 20, der aus reinem Trägergas 15 besteht, wird in einem Wärmeaustauscher 21 überhitzt, d.h. auf eine Temperatur gebracht, die über der Dehydrierungstemperatur liegt, und ebenfalls in den Reaktionsraum 19 geleitet.
Der Reaktionsraum 19 wird durch ein Rohr mit der Länge 200-450 mm, Innendurchmesser 4-21 mm gebildet. Die Produktgase werden in einem Kühler 22 nach Austritt aus dem Reaktionsraum 19 rasch auf eine Temperatur kleiner 200 °C gekühlt und mittels eines Gaschromatographen analysiert. In einer Kolonne 23 werden die Reaktionsprodukte mit Alkohol 24 (z.B. Cyclohexanol bei 20-80 °C) gewaschen, um den Formaldehyd 25 auszutragen. Als Primärkatalysator wird Natriummethylat verwendet, als Trägergas $H_2$/CO oder Stickstoff. Der Gesamtstrom beträgt 20-500 l/h. Die Methanolzufuhr ist so bemessen, daß sich eine Methanol-Konzentration von ca. 5-20 mol-% einstellt.
Die angegebenen Meßgrößen werden wie folgt berechnet:

$$\text{Ausbeute (in \%)} = \frac{\text{gebildeter Formaldehyd (mol)}}{\text{zudosiertes Methanol (mol)}} \cdot 100$$

A. Einfluß der Temperaturführung

| | Beispiel 1 | Vergleichsbeispiel 1 | Vergleichsbeispiel 2 | Vergleichsbeispiel 3 | Beispiel |
|---|---|---|---|---|---|
| Ofentemperatur Katalysatorzersetzung | 900°C | 900°C | 750°C | 750°C | 950°C |
| Ofentemperatur Reaktor | 750°C | 900°C | 750°C | 900°C | 800°C |
| Ausbeute Formaldehyd | 72 % | 13% | 0,7% | 34 % | 78% |

B. Einfluß der Verweilzeiten (Verweilzeiten abhängig)

(Verweilzeiten bei Normalbedingungen)

| | Beispiel 1 | Beispiel 2 |
|---|---|---|
| Verweilzeit Katalysatorzersetzung | 0,6 sec | 1,2 sec |
| Verweilzeit im Reaktor | 1,1 sec | 2,2 sec |
| Ausbeute Formaldehyd | 72 % | 67 % |

C. Einfluß der Verweilzeiten (Verweilzeiten unabhängig)

(Verweilzeiten bei Normalbedingungen)

| Reihe 1 | Beispiel 1 | Beispiel 2 | Beispiel 3 |
|---|---|---|---|
| Verweilzeit Katalysatorzersetzung | 0,6 sec | 1,2 sec | 0,6 sec |
| Verweilzeit im Reaktor | 1,1 sec | 1,1 sec | 0,55 sec |
| Ausbeute Formaldehyd | 72 % | 77 % | 63 % |

(Verweilzeit bei Normalbedingungen)

| Reihe 2 | Beispiel 4 | Beispiel 5 |
|---|---|---|
| Verweilzeit Katalysatorzersetzung | 0,4 | 0,4 |
| Verweilzeit im Reaktor | 0,7 | 0,4 |
| Ausbeute Formaldehyd | 68 % | 78 % |

## Patentansprüche

1. Verfahren zur Herstellung von Formaldehyd aus Methanol durch Dehydrierung in einem Reaktor in Gegenwart eines Katalysators bei einer Temperatur im Bereich von 300 bis 1000°C, **dadurch gekennzeichnet, daß** man die Erzeugung des Katalysators räumlich getrennt vom Reaktor und bei einer Temperatur oberhalb der Dehydrierungstemperatur durchführt.

2. Verfahren nach Anspruch 1, **dadurch gekennzeichnet, daß** die Temperaturdifferenz zwischen der Katalysatorerzeugungs- und der Dehydrierungstemperatur mindestens 20°C beträgt.

3. Verfahren nach Anspruch 1 oder 2, **dadurch gekennzeichnet, daß** dem Reaktor ein Trägergasstrom zugeführt wird, der eine Temperatur aufweist, die ungleich der Dehydrierungstemperatur ist.

4. Verfahren nach einem oder mehreren der vorhergehenden Ansprüche, **dadurch gekennzeichnet, daß** man einen Primärkatalysator zur Erzeugung des Katalysators verwendet und dieser Primärkatalysator, vorzugsweise kontinuierlich nachgeführt wird.

5. Verfahren nach einem oder mehreren der vorhergehenden Ansprüche, **dadurch gekennzeichnet, daß** das Trägergas im wesentlichen aus den Nebenprodukten der Dehydrierung besteht.

6. Verfahren nach einem oder mehreren der vorhergehenden Ansprüche, **dadurch gekennzeichnet, daß** man einen Teil der Nebenprodukte der Dehydrierung als Brennstoff zur Beheizung des Reaktors verwendet.

7. Vorrichtung zur Durchführung eines Verfahrens gemäß einem oder mehreren der vorhergehenden Ansprüche, enthaltend einen oder mehrere Wärmeaustauscher zum Vorwärmen der Ausgangsstoffe, einen beheizten Behälter zur Zersetzung eines Primärkatalysators, einen beheizten Reaktor zur Durchführung der Dehydrierung, einen oder mehrere Wärmeaustauscher zum Abkühlen des Produktgemisches, eine Einrichtung zum Abtrennen des. Formaldehyds, eine Vorrichtung zum Einbringen des Methanols und zur Nachführung des Primärkatalysators, sowie Mittel zur Einstellung unterschiedlicher Temperaturen in dem Behälter zur Zersetzung des Primärkatalysators und dem Reaktor.

8. Verfahren nach einem oder mehreren der Ansprüche 1 bis 6, **dadurch gekennzeichnet, daß** man als Nebenprodukt anfallenden Wasserstoff abtrennt und einer weiteren Verwertung zuführt.

**9.** Verfahren zur Herstellung von Trioxan, **dadurch gekennzeichnet, daß** man Formaldehyd nach einem Verfahren gemäß einem oder mehreren der Ansprüche 1 bis 6 und 8 herstellt, und

- den so hergestellten Formaldehyd zu Trioxan trimerisiert.

**10.** Verfahren zur Herstellung von Polyoxymethylen, **dadurch gekennzeichnet, daß** man Formaldehyd nach einem Verfahren gemäß einem oder mehreren der Ansprüche 1 bis 6 und 8 herstellt, und

- den so gewonnen Formaldehyd gegebenenfalls reinigt,
- den Formaldehyd polymerisiert,
- die Endgruppen des so hergestellten Polymers absättigt (Capping) und
- gegebenenfalls das Polymer in der Schmelze homogenisiert und/oder mit geeigneten Zusätzen versieht.

**11.** Verfahren zur Herstellung von Polyoxymethylen-Copolymeren, **dadurch gekennzeichnet, daß** man Formaldehyd nach einem Verfahren gemäß einem oder mehreren der Ansprüche 1 bis 6 und 8 herstellt, und

- den so gewonnenen Formaldehyd zu Trioxan trimerisiert,
- gegebenenfalls das Trioxan reinigt,
- das Trioxan mit cyclischen Ethern oder cyclischen Acetalen copolymerisiert,
- gegebenenfalls instabile Endgruppen entfernt und
- das so hergestellte Copolymer gegebenenfalls in der Schmelze homogenisiert und/oder mit geeigneten Zusatzstoffen versetzt.

**12.** Verfahren zur Herstellung von Polyoxymethylen-Copolymeren, **dadurch gekennzeichnet, daß** man Formaldehyd nach einem Verfahren gemäß einem oder mehreren der Ansprüche 1 bis 6 und 8 herstellt, und

- den so gewonnenen Formaldehyd gegebenenfalls reinigt,
- den Formaldehyd mit cyclischen Ethern oder cyclischen Acetalen copolymerisiert,
- gegebenenfalls instabile Endgruppen entfernt und
- das so hergestellte Polymer gegebenenfalls in der Schmelze homogenisiert und/oder mit geeigneten Zusatzstoffen versetzt.

## Claims

**1.** A process for preparing formaldehyde from methanol by dehydrogenation in a reactor in the presence of a catalyst at a temperature in the range from 300 to 1000°C, wherein the catalyst is generated spatially separately from the reactor and at a temperature above the dehydrogenation temperature.

**2.** The process as claimed in claim 1, wherein the temperature difference between the catalyst generation and the dehydrogenation temperature is at least 20°C.

**3.** The process as claimed in claim 1 or 2, wherein a carrier gas stream having a temperature which is not the same as the dehydrogenation temperature is fed to the reactor.

**4.** The process as claimed in one or more of the preceding claims, wherein a primary catalyst is used for generating the catalyst and further amounts of this primary catalyst are fed in, preferably continuously.

**5.** The process as claimed in one or more of the preceding claims, wherein the carrier gas consists essentially of the by-products of the dehydrogenation.

**6.** The process as claimed in one or more of the preceding claims, wherein part of the by-product of the dehydrogenation is used as fuel for heating the reactor.

**7.** An apparatus for carrying out a process as claimed in one or more of the preceding claims comprising one or more heat exchangers for preheating the starting materials, a heated vessel for decomposing a primary catalyst, a heated reactor for carrying out the dehydrogenation, one or more heat exchangers for cooling the product mixture, a unit for separating off the formaldehyde, an apparatus for introducing the methanol and for feeding in further

primary catalyst and also means of setting different temperatures in the vessel for decomposing the primary catalyst and in the reactor.

**8.** The process as claimed in one or more of claims 1 to 6, wherein the hydrogen obtained as by-product is separated off and passed to a further use.

**9.** A process for preparing trioxane, which comprises preparing formaldehyde by a process as claimed in one or more of claims 1 to 6 and 8, and

- trimerizing the formaldehyde prepared in this way to give trioxane.

**10.** A process for preparing polyoxymethylene, which comprises preparing formaldehyde by a process as claimed in one or more of claims 1 to 6 and 8, and

- if desired, purifying the formaldehyde obtained in this way,
- polymerizing the formaldehyde,
- capping the end groups of the polymer prepared in this way and
- if desired, homogenizing the polymer in the melt and/or providing it with suitable additives.

**11.** A process for preparing polyoxymethylene copolymers, which comprises preparing formaldehyde by a process as claimed in one or more of claims 1 to 6 and 8, and

- trimerizing the formaldehyde obtained in this way to give trioxane,
- if desired, purifying the trioxane,
- copolymerizing the trioxane with cyclic ethers or cyclic acetals,
- if desired, removing unstable end groups and
- if desired, homogenizing the copolymer prepared in this way in the melt and/or admixing is with suitable additives.

**12.** A process for preparing polyoxymethylene copolymers, which comprises preparing formaldehyde by a process as claimed in one or more of claims 1 to 6 and 8, and

- if desired, purifying the formaldehyde obtained in this way,
- copolymerizing the formaldehyde with cyclic ethers or cyclic acetals,
- if desired, removing unstable end groups and
- if desired, homogenizing the polymer prepared in this way in the melt and/or admixing it with suitable additives.

**Revendications**

**1.** Procédé pour la préparation de formaldéhyde à partir de méthanol par déshydratation dans un réacteur en présence d'un catalyseur à une température dans la plage de 300 à 1000°C, **caractérisé en ce qu'**on réalise la production du catalyseur de manière séparée dans l'espace du réacteur et à une température supérieure à la température de déshydratation.

**2.** Procédé selon la revendication 1, **caractérisé en ce que** la différence de température entre la température de production du catalyseur et la température de déshydratation est d'au moins 20°C.

**3.** Procédé selon la revendication 1 ou 2, **caractérisé en ce qu'**on introduit dans le réacteur un flux de gaz porteur qui présente une température qui est différente de la température de déshydratation.

**4.** Procédé selon l'une ou plusieurs des revendications précédentes, **caractérisé en ce qu'**on utilise un catalyseur primaire pour la production du catalyseur et que ce catalyseur primaire est de préférence ajusté en continu.

**5.** Procédé selon l'une ou plusieurs des revendications précédentes, **caractérisé en ce que** le gaz porteur est essentiellement constitué des produits secondaires de la déshydratation.

**6.** Procédé selon l'une ou plusieurs des revendications précédentes, **caractérisé en ce qu'**on utilise une partie des

produits secondaires de la déshydratation comme combustible pour le chauffage du réacteur.

**7.** Dispositif pour réaliser un procédé selon l'une ou plusieurs des revendications précédentes, contenant un ou plusieurs échangeurs de chaleur pour le préchauffage des matières de départ, un récipient chauffé pour la décomposition d'un catalyseur primaire, un réacteur chauffé pour réaliser la déshydratation, un ou plusieurs échangeurs de chaleur pour le refroidissement du mélange de produits, un dispositif pour séparer le formaldéhyde, un dispositif pour l'introduction du méthanol et pour l'ajustement du catalyseur primaire, ainsi que des moyens pour le réglage de températures différentes dans le récipient pour la décomposition du catalyseur primaire et le réacteur.

**8.** Procédé selon l'une ou plusieurs des revendications 1 à 6, **caractérisé en ce qu'**on sépare de l'hydrogène produit comme produit secondaire et qu'on l'introduit dans une valorisation ultérieure.

**9.** Procédé pour la préparation de trioxanne, **caractérisé en ce qu'**on prépare du formaldéhyde selon un procédé selon l'une ou plusieurs des revendications 1 à 6 et on trimérise le formaldéhyde ainsi préparé en trioxanne.

**10.** Procédé pour la préparation de polyoxyméthylène, **caractérisé en ce qu'**on prépare du formaldéhyde selon un procédé selon l'une ou plusieurs des revendications 1 à 6 et 8 et

- on purifie le cas échéant le formaldéhyde ainsi obtenu,
- on polymérise le formaldéhyde,
- on sature les groupements terminaux du polymère ainsi préparé (Capping) et
- on homogénéise le cas échéant le polymère dans la masse fondue et/ou on le pourvoit des additifs appropriés.

**11.** Procédé pour la préparation de copolymères de polyoxyméthylène, **caractérisé en ce qu'**on prépare du formaldéhyde selon un procédé selon l'une ou plusieurs des revendications 1 à 6 et

- on trimérise le formaldéhyde ainsi obtenu en trioxanne,
- on purifie le cas échéant le trioxanne
- on copolymérise le trioxanne avec des éthers cycliques ou des acétals cycliques,
- on élimine le cas échéant des groupements terminaux instables, et
- on homogénéise le cas échéant le copolymère ainsi préparé dans la masse fondue et/ou on le mélange avec des additifs appropriés.

**12.** Procédé pour la préparation de copolymères de polyoxyméthylène, **caractérisé en ce qu'**on prépare du formaldéhyde selon un procédé selon l'une ou plusieurs des revendications 1 à 6 et 8 et

- on purifie le cas échéant le formaldéhyde ainsi obtenu,
- on copolymérise le formaldéhyde avec des éthers cycliques ou des acétals cycliques,
- on élimine le cas échéant des groupements terminaux instables, et
- on homogénéise le cas échéant le copolymère ainsi préparé dans la masse fondue et/ou on le mélange avec des additifs appropriés.

Fig. 1

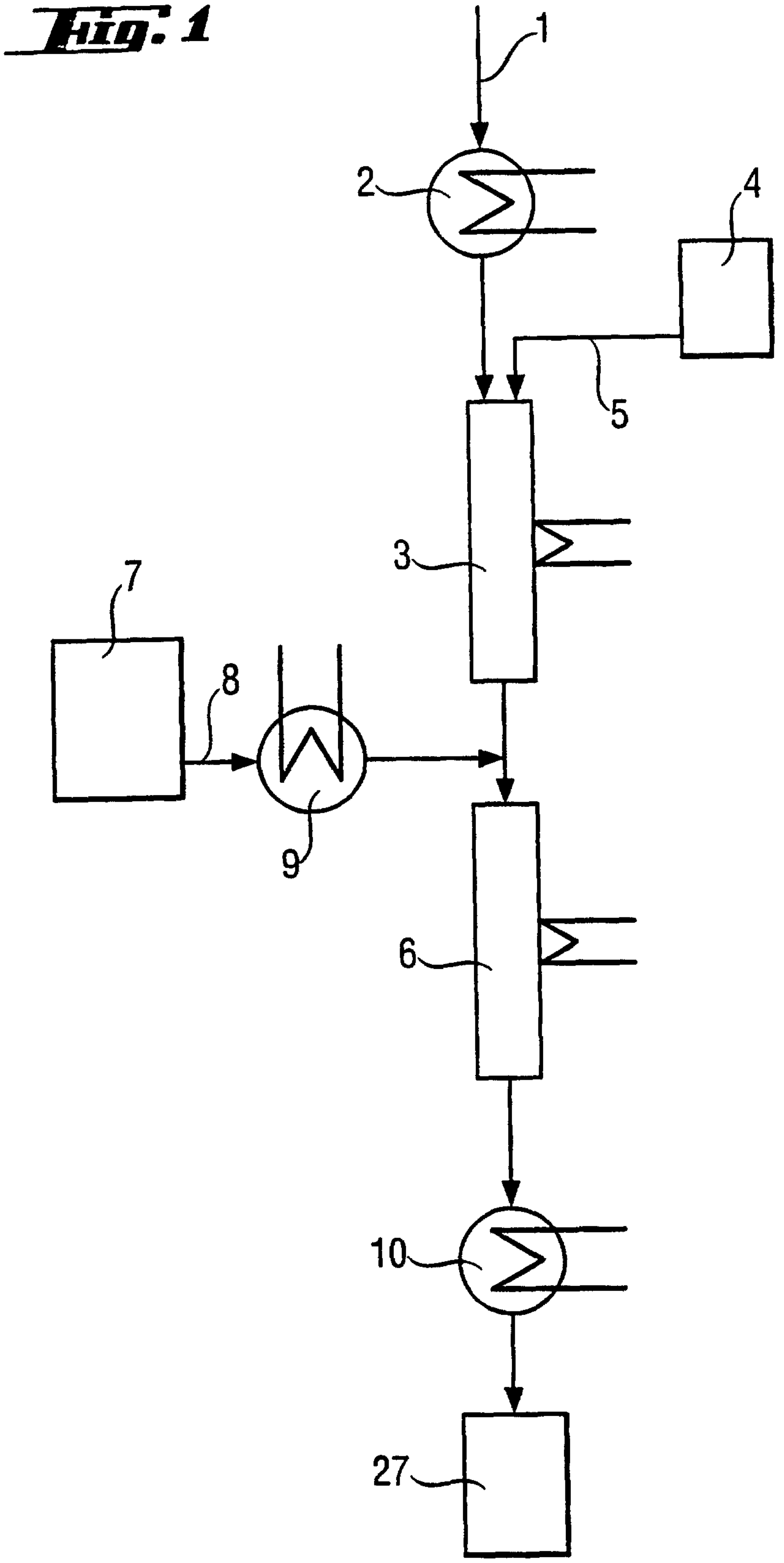

Fig. 2

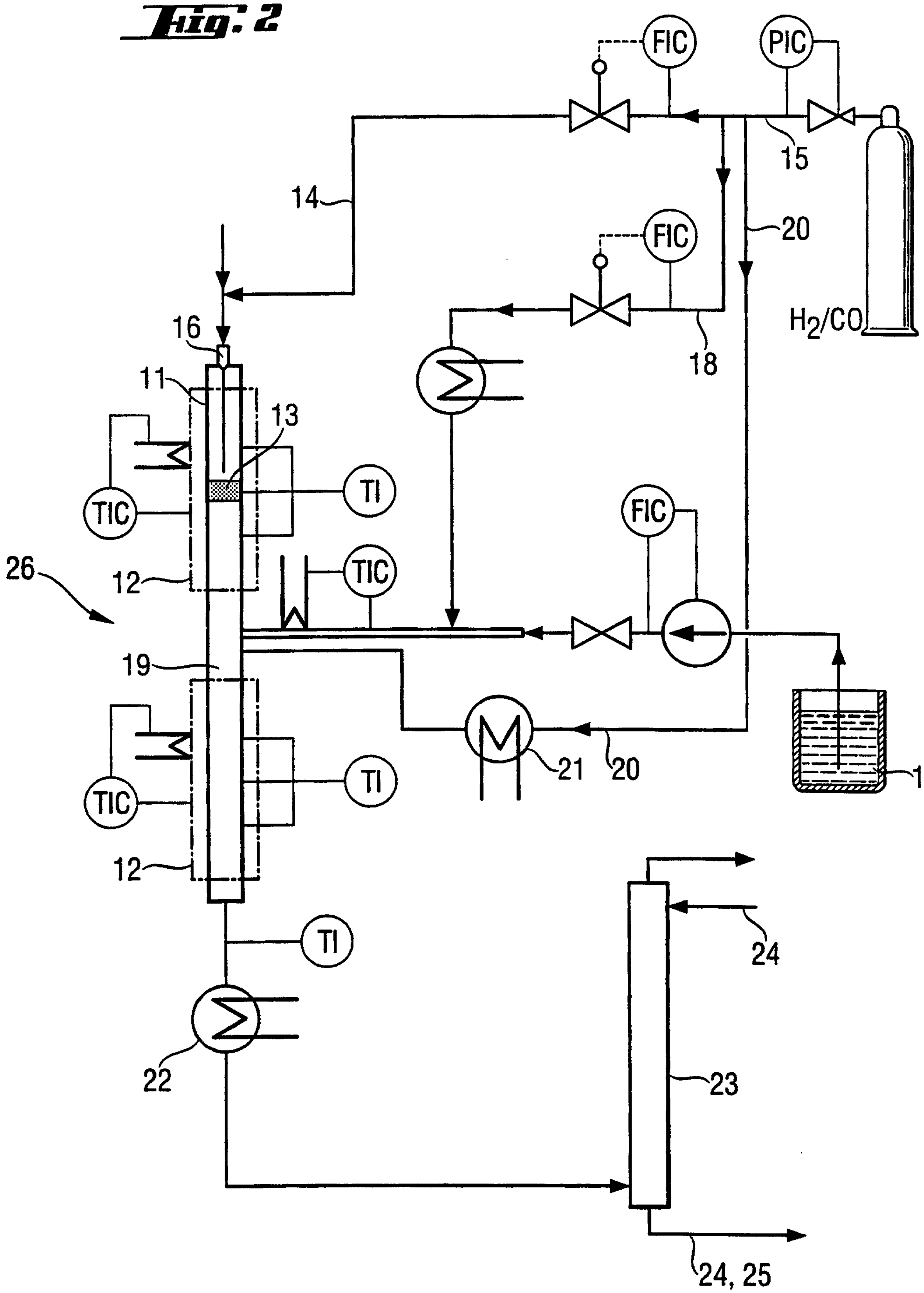
FIC
PIC
FIC
FIC
14
15
20
16
18
H2/CO
11
13
TI
TIC
TIC
26
12
19
21
20
17
TIC
TI
12
TI
24
22
23
24, 25